# EUROPEAN PATENT SPECIFICATION

(11) **EP 2 838 493 B1**
(45) Date of publication and mention of the grant of the patent: **01.11.2017**
(21) Application number: 13714957.1
(22) Date of filing: 10.04.2013
(51) Int. Cl.: A61K 8/31, A61K 8/34, A61K 8/36, A61K 8/37, A61Q 19/00

(54) **PRE-BLENDED MIXTURES OF SPECIFIC HYDROCARBON LIQUIDS STRUCTURED WITH HIGH MELTING POINT STRUCTURING MATERIAL**
MIT STRUKTURIERUNGSMATERIAL MIT HOHEM SCHMELZPUNKT STRUKTURIERTE VORGEMISCHTE MISCHUNGEN AUS SPEZIFISCHEN KOHLENWASSERSTOFFFLÜSSIGKEITEN
MÉLANGES PRÉ-MÉLANGÉS DE LIQUIDES HYDROCARBONÉS SPÉCIFIQUES, STRUCTURÉS À L'AIDE D'UNE MATIÈRE DE STRUCTURATION À POINT DE FUSION ÉLEVÉ

(30) Priority: 16.04.2012 US 201213447343
(43) Date of publication of application: 25.02.2015
(73) Proprietor: Unilever PLC, London, Greater London EC4Y 0DY (GB); Unilever N.V., 3013 AL Rotterdam (NL)
(72) Inventor: LIU, Hongjie, Trumbull, Connecticut 06611 (US); HU, Yuntao, Thomas, The Woodlands, Texas 77381 (US); CHANDAR, Prem, Trumbull, Connecticut 06611 (US)
(74) Representative: Fijnvandraat, Arnoldus
(86) International application number: PCT/EP2013/057510
(87) International publication number: WO 2013/156370

(56) References cited:
- WO-A2-2004/066918
- WO-A2-2008/114190
- CA-A1- 2 615 024
- US-A1- 2003 207 971
- US-B1- 6 258 346

## Description

### FIELD OF THE INVENTION

The present invention relates to novel pre-blended mixtures of specific hydrocarbon liquids of defined viscosity structured with high melting point materials, e.g., waxes. These pre-blends provide occlusivity (e.g., moisture retention) which approaches, equals or surpasses that of petrolatum, an objective not easily obtained. In this regard, the materials can be used as substitutes for petrolatum. The mixtures can be used as stand-alone blends for use in a variety of personal care compositions (e.g., personal wash, hair care) and/or they can be blended in separate mixers as part of overall process when making the above-noted products. In applicants' separately filed co-pending application, applicants claim mixtures wherein the base hydrocarbon liquids and the structurants are derived specifically from naturally sourced materials (by naturally sourced, we refer to non-petrolatum based materials).

### BACKGROUND

Petrolatum is a colorless or pale mellow semi-solid material comprising a mixture of hydrocarbons and which is often used in cosmetic and personal care products (e.g., cleansing products, skin-care products, make-up, shampoos and conditioners, shaving products, suntan products). For example, it can function as hair conditioning agent, skin protectant, occlusive skin-conditioning agent, etc.

It is extremely difficult to find alternative products which are similar to and/or function equivalently to petrolatum. Unexpectedly, applicants have now found materials (structured, pre-blended mixtures) and methods (i.e., formation of said pre-mixtures) for approaching, matching or surpassing some of the properties (e.g., occlusivity) of petrolatum while retaining rheological characteristics. The present invention is directed to all pre-blends, in which hydrocarbon liquids (used as base of the pre-blend) may be petroleum or non-petroleum materials. In a related co-pending application, applicants claim more specifically pre-blends in which both the hydrocarbon base liquids and the strucuturants are all natural ingredients (non-petrolatum derived).

As noted, applicants have unexpectedly now found that a pre-blend of specific hydrocarbon liquids (e.g., low molecular weight alkanes, oligomers and polymers) of defined viscosity and defined melting or glass transition point, together with structuring materials of defined melting point, can be used instead of petrolatum (e.g., petrolatum jelly or "PJ"); and will approach, match or surpass the occlusivity of PJ, while also providing spreadability required to achieve the film forming and sensory profile desired by consumers.

Various references disclose compositions where waxes, for example, are disclosed and/or polymers/oligomers are disclosed; but no reference of which applicants are aware discloses a pre-blend where specific hydrocarbons (i.e., specifically selected to have melting or glass transition point below 30°C; and a viscosity of 500 Pa.s or below at room temperature) are pre-blended with specific high melting point materials, either for use as separate component (stand-alone blends for use as ingredients) or, optionally, before combining with other compositional ingredients during preparation of personal care compositions.

US2003/207971 relates an emollient gel useful as an ingredient in inks, paint, lubricants, grease and cosmetics, and particularly as a replacement for petrolatum in such applications. The emollient gel is composed of 40-98 percent of an oil, or blend of oils, and 2-60 percent of a thickening wax, or blend of thickening waxes.

U.S. Patent No. 5,869,070 and U.S. Patent No. 6,033,680, both to Dixon et al., disclose dual cleanser moisturizer compositions which may comprise mixtures of petrolatum with the types of oil used in our invention (e.g., polybutene) at defined ratios of petrolatum to the hydrocarbon oils (col. 7, lines 5-12); and/or where wax alone is disclosed (e.g., col. 6, line 60), but nowhere is there disclosed mixture (particularly where pre-blended) of wax or other high melting point ingredients and hydrocarbon liquids to form the structured pre-blended mixtures of our invention.

WO 99/09950 to Elliott et al. (P&G) discloses the use of certain branched polymers, as well as, broadly, possible use of waxes. However, nowhere is there recognized specifically preparing a pre-blend to obtain the materials disclosed in our invention.

U.S. Patent No. 6,521,573 to Tsaur et al. also discloses generally the use of hydrocarbon polymers and hydrophobic oils. There is no mention of wax and certainly no suggestion of pre-blending specifically selected hydrocarbon liquids (having viscosity of 500 Pa.s or below and melting or glass transition point of <30°C); and high melting point structuring materials to produce the pre-blends of our invention.

U.S. Patent No. 7,776,346 to O'Connor et al. discloses oils which may be structured with certain crystalline materials. There is no disclosure of the specific polymers/oligomers of our invention pre-blended with high melting point materials to unexpectedly obtain pre-blends which approach, match or surpass occlusivity, while retaining rheological characteristics (spreadability, feel) of petrolatum.
In short, applicants are aware of no references which teach or suggest the unique hydrocarbon liquids and the structuring materials of our invention pre-blended to form the mixtures of our invention, i.e., mixtures which provide the unexpected advantage of approaching, matching or surpassing occlusivity, while retaining rheological characteristics (spreadability) of petrolatum. Petrolatum is a very unique material and obtaining functional equivalents is not a readily achievable goal.

### BRIEF DESCRIPTION OF THE INVENTION

In one embodiment, the present invention relates to specific premix compositions which provide an occlusivity which approaches, equals or surpasses that of petrolatum while simultaneously retaining rheological characteristics (spread, feel) of petrolatum.

These pre-blends are made by mixing a base or continuous phase comprising specific hydrocarbon liquids with specific high melting point structuring agents. Examples of materials which can be used as (1) the base hydrocarbon liquid phase and (2) as the structuring agent are summarized below.

| Continuous Phase | Structuring Agents |
|---|---|
| Polybutene,Ca | ndelilla wax |
| natural rubber and | |
| mixtures of the above materials. | |

More specifically, pre-blended mixture compositions of the invention comprise:
(a) 25 to 95%, preferably 40 to 90% by wt. of hydrocarbon liquids being petroleum-derived oligomers and polymers comprising alkene monomeric groups or contain two or more alkene units, wherein the hydrocarbons of (a) have a melting or glass transition point <30°C such that they are flowable (viscosity 500 Pa.s or below) at room temperature (about 20-25°C), and wherein the hydrocarbon liquids are polybutene or natural rubber and
(b) 10 to 75%, preferably 10 to 60% by wt. of structuring materials for the hydrocarbon liquids selected from the group consisting of waxes, wherein these materials have melting point above 30°C, preferably above 35°C and more preferably above 45°C; wherein the wax is Candelilla wax,
wherein the pre-blended mixtures have viscosity from 1 Pa.s to 1000 Pa.s, preferably 5 to 500 Pa.s when measured at shear rate of 10s⁻¹ at 25 °C; and an occlusivity of greater than 80, preferably greater than 90, more preferably equal to or greater than 99.

Even when occlusivity of blend is lower than petrolatum (e.g., 99 or 105), the occlusivity is 50% or more, preferably 100% or more, more preferably 150% or more and even more preferably 200% or more (i.e., two times or more) higher in occlusivity compares to the base alone.

Claims of the subject invention are directed to petroleum or non-petroleum derived materials as both hydrocarbon liquid base phase and structurant, while claims of co-pending application are specifically directed to non-petroleum derived base and structuring materials.

When hydrocarbon liquids of the invention are pre-blended with structuring materials in a pre-mix, they approach, match or surpass the intrinsic occlusivity of petrolatum (i.e., are excellent moisturizers) while also retaining the rheology (e.g., spreadability) of petrolatum. It is an unexpected advantage to produce such blends using any materials. It is particularly advantageous to make such materials from so-called natural materials (i.e., non-petroleum derived). Blends prepared from these specific natural materials (for both base and structuring material) are claimed in a related co-pending application.

In a second embodiment, the invention relates to a method of obtaining pre-blended materials approaching, matching or surpassing occlusivity of petrolatum while retaining rheology of petrolatum which method comprises pre-blending specifically selected hydrocarbon liquids with specific high melting point structuring materials to form pre-blends of defined viscosity as defined above.

In a third embodiment, the invention relates to a method of providing petrolatum-like rheology and moisturized feel (to people in need of such moisturized feel) to skin or other desired substrate which method comprises applying to such skin or substrate specific combination of ingredients noted above which have been pre-blended. The pre-blends may be applied directly or, optionally, mixed into personal care compositions (subsequent to having been specifically prepared as a pre-blend).

In the table in the Examples, applicants show 6 examples of pre-blend compositions of hydrocarbon liquid bases and structuring agents. The occlusivities of the pre-blends prepared by the process of the invention are much higher than those of the bases alone or those of simple blends made by merely combining ingredients; and the occlusivities approach, match or surpass the occlusivity of petrolatum.

Specifically, Blend 1 in the Table shows that structuring commercial hydrocarbon polybutene (Parapol® 950 Exxon Mobil) with Candelilla wax increases occlusivity of Parapol® 950 to the level of petrolatum.

Blend 2 matched the occlusivity of petrolatum when structuring naturally occurring hydrocarbon-natural rubber (polyisoprene) with Candelilla wax.

Blend 3 shows a less viscous polybutene (viscosity 1.0 Pa.s at 25 °C) structured with high concentration of Candelilla wax, and wherein occlusivity of the blend is both much higher than that of the base alone, and approaches the occlusivity of petrolatum.

Blends 4, 5 and 6 in the Table are additional examples of pre-blended mixtures having an occlusivity which approaches, matches or surpasses that of petrolatum.

### DETAILED DESCRIPTION OF THE INVENTION

In one embodiment, the present invention relates to novel, unique pre-blended materials which sallow for use of specific structured, hydrocarbon liquids. These pre-blended mixtures approach, equal or surpass the moisturizing properties (occlusivity) and simultaneously retain rheological properties (spreadability) of petrolatum.
Specifically, the premix is made by blending the following two materials:
(a) 25 to 95%, preferably 25 to 90% by wt. specific hydrocarbon liquids such as oligomers and polymers (specific non-petroleum materials are claimed in co-pending application);
   wherein the hydrocarbon liquids of (a) have a melting or glass transition point <30°C such that they are flowable; by flowable is meant they have viscosity of 500 Pa.s or below at room temperature; and
(b) 10 to 75%, preferably 10 to 60% by wt. of structurants for the hydrocarbon liquids selected from the group consisting of waxes, wherein the structuring materials have a melting point above 30°C, preferably above 35°C, and more preferably above 45°C;
wherein the pre-blended mixtures have viscosity of 1 to 1000 Pa.s, preferably 5 to 500 Pa.s, measured at shear rate of 10s⁻¹ at 25 °C and an occlusivity of greater than 80, preferably greater than 90, more preferably equal to or greater than 99. Further occlusivity of blend is 50% or more, preferably 100% or more, more preferably 150% or more, and even more preferably 200% or more greater than occlusivity of base alone.

### Hydrocarbon liquids

The hydrocarbon liquids of the invention are typically, but not necessarily, petroleum derived materials (natural, non-petroleum derived oils are specifically claimed in co-pending application).

In order to make a spreadable pre-blend using the solid, high melting point structuring ingredients of our invention, the hydrocarbon materials used as the base or continuous phase of our invention must be in liquid form at room temperature. Materials used include, but are not limited to, low molecular weight alkanes, certain oligomers and polymers and their mixtures. The oligomers and/or polymers typically comprise at least one alkene monomer (examples of such materials include polybutene or polyisobutylene). The molecule may further comprise two or more alkene units as, for example, polybutene diene. Other preferred molecules include two or more isoprene units such as, for example, polyisoprene, terpene, squalene. Saturated hydrocarbon-containing branched molecules (e.g., squalane) can also be used. What is important is that the molecule be flowable around room temperature, i.e., has viscosity of 500 Pa.s or less.

As noted, the material must be flowable. Specifically, material must have melting or glass transition point <30°C such that they are flowable at room temperature (measured at 20-25°C) with the viscosity at room temperature of 500 Pa.s or below.

The hydrocarbon liquids comprise 25 to 95%, preferably 40 to 90% by wt. of the pre-blend.

### Structuring Materials

The structuring materials of the invention may comprise waxes; hydrogenated triglycerides; long chain (C₁₄-C₂₄ preferably C₁₆-C₂₄) fatty acids; long chain fatty alcohols and esters; wherein melting point of said structuring material is greater than 30°C, preferably greater than 45°C.

Among waxes which may be used are animal waxes, such as for example beeswax. Other animal waxes include Chinese wax (produced by ceroplastes ceriferus), earwax, lanolin, shellac wax and spermaceti.

Also included are various plant and vegetable waxes such as Candelilla wax, bayberry wax, carnauba wax, castor wax (e.g., hydrogenated castor oil), Japan wax (a vegetable triglyceride), jojoba oil, ouricury wax, rice bran wax and soy wax. Preferred waxes include soy wax and Candelilla wax.

Other materials which may be used as structuring material include animal fat (tallow) and, as noted, long chain fatty acids, fatty alcohols and esters.

Specific examples of premix blends include high viscous hydrocarbons such as polybutene or rubber mixed with, for example, relatively low level (e.g., 20% by wt.) of a wax; or low viscous hydrocarbon (e.g., squalane, mineral oils) with relatively high level (e.g., 50% by wt.) of a wax. It should be noted that if levels of structurant become too high (e.g., above 75%), the spreadability/rheology of the blend may become readily compromised. A range of 20 to 50% structuring material is particularly preferred.

### Pre-blend Processing

A further critical aspect of the invention is that the hydrocarbon liquids and structuring materials must be pre-blended. Pre-blends are prepared by mixing all the ingredients together at temperature 10-20°C higher than the highest melting point of any ingredient being blended (e.g., typically this is the structuring material such as wax or fat), typically about 80°C. The pre-blends are mixed (e.g., with overhead mixer) at speed of about 500-1500 revolutions per minute (rpm) for about 20 to 60, preferably 20 to 40 minutes until completely mixed. Heating is discontinued and mixing is continued until pre-blends are cool at room temperature.

The pre-blended mixture of hydrocarbon liquids and structuring materials needs to be mixed by the claimed process in order to provide advantageous properties. The non pre-blended mixture of those materials is not homogeneous and could not even form a uniform film for measurement, let alone obtain the claimed benefit.

### Composition

The pre-blended mixtures of the invention may be used, optionally, in personal wash or hair care compositions. For examples, they may be used in a liquid composition comprising:
1) 0 to 99%, preferably 1 to 75%, more preferably 3 to 50% by wt. of a surfactant (e.g., anionic, amphoteric, nonionic or cationic surfactant and mixtures thereof);
2) optional ingredients typically found in liquid personal cleanser for skin or hair; and
3) balance water.

Optional ingredients in personal wash cleanser may include oils (e.g., vegetable oils such as castor oil); esters such as cetyl palmitate; animal fats such as lanolin; fatty acids and alcohols; and other oils-emollients such as mineral oils petrolatum, silicone oil.

Further may be included fatty acids which help form lamellar phase, as well as cationic conditioning agent.

Other optional ingredients which may be used in liquid cleansers include organic solvents (e.g., ethanol); thickeners (e.g., carboxymethyl cellulose); perfumes; sequestering agents; opacifiers; pearlizers; antimicrobials; suds booster (e.g., alkanolamides); antioxidants; thickeners; exfolliants, etc.

Hair compositions may be, for example, shampoos or conditioners. Shampoos may further comprise, in addition to surfactant, cationic polymers; suspending agents (e.g., polyacrylic acids). Conditioners may comprise cationic conditioning surfactants; silicone conditioning agents, fragrances, dyes, pigments, pH adjustment agents, pearlecers, viscosity modifiers, preservatives and antimicrobials.

In another embodiment, the invention relates to method of obtaining premixtures having or surpassing occlusivity of petrolatum while maintaining applicable rheology property (e.g., spreadability, feel) which method comprises pre-blending specific hydrocarbon liquids (as defined above) of defined viscosity with specific concentration of high melting point materials also defined above.

In a third embodiment, the invention relates to a method of providing moisturization (e.g., occlusivity) to skin or other substrate, while retaining rheology of petrolatum, which method comprises applying to substrate a pre-blend of specific hydrocarbon liquids and structuring material noted above either directly as pre-blend product or as composition comprising said pre-blend.

### Protocol

We define water occlusivity as the inverse of water flux through the occlusive film for a dosage of 1 g/cm², and as having the unit of (g/m² hr)⁻¹. The water occlusivity of pre-blends was measured using the AquaFlux® evaporimeter from Biox (LSBU). The pre-blends were dosed onto a porous supporting membrane to form a film. The equilibrium water (vapor) fluxes for different dosage were measured (TEWL reading). According to Fick's law, the reverse of water flux linearly depends on the film thickness (also dose for the same material) and the numerical value of the slope represents the average occlusivity.

The spreadability was evaluated by finger/hand palm rubbing of measured by viscometer using the standard methods as defined (ASTM D445, D2270, D937, D1321).

### Examples 1-6

Table 1 below shows six examples of pre-blends of the invention. The pre-blends comprise the base (continuous phase) and a structuring agent. The occlusivities of the blends are much higher than those of the bases alone and some of them approach, match or surpass the occlusivity of petrolatum.

**Table 1**

| | **base/continuous phase** | **structuring agent** | **occlusivity of base (gram/m².hr)⁻¹** | **occlusivity of blend (gram/m².hr)⁻¹** |
|---|---|---|---|---|
| **Petrolatum** | - | - | - | 148±44 |
| **Blend 1** | 80%PARAPOL® 950 | 20%Candelilla wax | 63±16 | 160±55 |
| **Blend 2** | 80%Natural Rubber | 20%Candelilla wax | 30±2 | 154±30 |
| **Blend 3** | 50%polybutehe (1 Pa.s) | 50%Candelilla wax | 5±1 | 105±17 |
| **Blend 4** | 80%polybutene (10Pa.s) | 20% Candelilla wax | 59±12 | 222+33 |
| **Comparative Blend 5** | 80%polybutene (76Pa.s) | 20% Hydrogenated soy | 79±24 | 128±22 |
| **Comparative Blend 6** | 50%Squalane | 50%Candelilla wax | 15±1 | 99±18 |

In blend 1, applicants prepared a pre-blend of polybutene Parapol® 950 (hydrocarbon liquid base) and Candellila wax (i.e. using 20% Candellila wax and 80% Parapol® 950). Parapol® 950 (ExxonMobil Chemical Company) is a liquid polybutene polymer having a molecular weight of 950 and a Viscosity of 30 Pa.s at room temperature. As seen from example blend 1 in Table 1, the occlusivity of the pre-blend is much higher than that of Parapol® 950 alone and surpasses that of petrolatum.

In blend 2, applicants prepared a pre-blend of 80% natural rubber (polyisoprene, DPR Industries, viscosity 40 Pa.s at 38 °C) structured with 20% Candelilla wax. As seen from blend 2 in Table 1, the occlusivity of the pre-blend is much higher than that of natural rubber alone and matches or surpasses that of petrolatum.

Applicants prepared pre-blends of polybutene hydrocarbon of different viscosities and Candelilla wax in blends 3 and 4. Blend 3 comprises less viscous polybutene (1 Pa.s at 25°C) and high level of structuring wax (50% Candelilla wax), and blend 4 comprises more viscous polybutene (10 Pa.s at 25°C) with 20% Candelilla wax. As seen from Table 1, the occlusivity of blend 3 approaches and occlusivity of blend 4 is significantly higher than that of petrolatum. Occlusivities of both are much higher than base alone.

In blend 5 applicants prepared a pre-blended mixture containing a higher viscosity polybutene hydrocarbon (76 Pa.s at 25°C) with 20% structuring wax (20% hydrogenated soy). As seen in Table 1, the occlusivity of blend approaches that of petrolatum.

In blend 6, applicants prepared a naturally sourced pre-blend containing 50% Squalane and 50% Candelilla wax. As seen in Table 1, the occlusivities of this natural pre-blend approaches that of petrolatum and is certainly much higher than base alone.

It is emphasized that all the pre-blends have a spreadable viscosity at room temperature. This is believed to be true at least in part because level of structurant is not too high (e.g., above 75%).

### Comparative Examples A-F

For blends of Comparative Examples A-F, applicants prepared mixtures of vegetable oils (e.g., soybean oil) and structuring agents. As seen in Table 2, the occlusivities of the pre-blends are much lower than that of petrolatum. These examples demonstrate that the selection of hydrocarbon base is critical to the occlusivity of the disclosed blends.

**Table 2**

| | **base/continuous phase** | **structuring agent** | **occlusivity of base** | **occlusivity of blend** |
|---|---|---|---|---|
| **Petrolatum** | - | - | - | 148±44 |
| **Comparative Blend A** | 75% soybean oil | 25% Hydrogenated soy | 6±2 | 16±1 |
| **Comparative Blend B** | 50% soybean oil | 50% Hydrogenated soy | 6±2 | 19±1 |
| **Comparative Blend C** | 20% soybean oil | 80% Hydrogenated soy | 6±2 | 61±8 |
| **Comparative Blend D** | 75% soybean oil | 25% Candelilla wax | 6±2 | 37+3 |
| **Comparative Blend E** | 50% soybean oil | 50% Candelilla wax | 6±2 | 27+5 |
| **Comparative Blend F** | 80% soybean oil | 20% Beeswax | 6±2 | 18+4 |

## Claims

1. A personal wash or hair care composition comprising a pre-mix composition, the pre-mix composition comprising:
(a) 25-95% by wt. of pre-blended mixture comprising hydrocarbon liquids being petroleum derived oligomers and polymers comprising alkene monomeric groups; or two or more alkene units; wherein said hydrocarbon liquids have a melting or glass transition point < 30°C and a viscosity of 500 Pa.s or less at 20 to 25 °C and wherein the hydrocarbon liquids are polybutene or natural rubber;
(b) 10 to 75% by wt. of pre-blended mixture comprising structuring material selected from the group consisting of waxes; wherein said structuring material has melting point > 30°C and wherein the wax is Candelilla wax,
wherein said pre-blend mixtures comprise flowable hydrocarbon liquid materials (Component a) structured with materials of b;
wherein the said pre-blends have viscosity below 1000 Pa.s measured at shear rate 10s⁻¹ at 25°C. and wherein said pre-blend mixtures are prepared by mixing (a) and (b) at 500-1500 rpm until completely mixed at temperature higher than highest melting point of any ingredient being blended and cooling to 20 to 25 °C to form pre-blend.

2. A composition according to claim 1, wherein the pre-blends have occlusivity of >80.

3. A composition according to any one of the preceding claims wherein pre-blend is mixed at temperature of 80°C for 20-60 minutes prior to cooling.

4. A method for obtaining pre-blend materials having occlusivity of greater than 80 and viscosity of <1000 Pa.s at 10 s⁻¹ at 25°C which comprises making a pre-blend as defined by components (a) and (b) of claim 1.

5. A method according to claim 4, wherein said pre-blend materials are prepared by mixing (a) and (b) at 500-1500 rpm until completely mixed at temperature higher than highest melting point of any ingredient being blended and cooling to 20 to 25 °C to form pre-blend.

6. A method of providing petrolatum like moisturization and sensory to skin or other substrate which comprises applying to substrate specific premix according to any one of claims 1 to 3.

## Patentansprüche

1. Körperwasch- oder Haarpflegezusammensetzung, die eine Vorgemisch-Zusammensetzung umfasst, wobei die Vorgemisch-Zusammensetzung umfasst:
(a) 25-95 Gew.-% des vorgemischten Gemischs, umfassend Kohlenwasserstoff-Flüssigkeiten, die von Petroleum abgeleitete Oligomere und Polymere, umfassend monomere Alken-Gruppen oder zwei oder mehrere Alken-Einheiten, darstellen, wobei die Kohlenwasserstoff-Flüssigkeiten einen Schmelz- oder Glasübergangspunkt < 30°C und eine Viskosität von 500 Pa.s oder weniger bei 20 bis 25°C aufweisen und wobei die Kohlenwasserstoff-Flüssigkeiten Polybuten oder Naturkautschuk darstellen,
(b) 10 bis 75 Gew.-% des vorgemischten Gemischs, umfassend Strukturierungsmaterial, das aus der aus Wachsen bestehenden Gruppe ausgewählt ist, wobei das Strukturierungsmaterial einen Schmelzpunkt > 30°C aufweist und wobei das Wachs Candelilla-Wachs darstellt,
wobei die vorgemischten Gemische fließfähige Kohlenwasserstoff-Flüssigmaterialien (Bestandteil a), strukturiert mit Materialien von b, umfassen, wobei die vorgemischten Gemische eine Viskosität unter 1000 Pa.s, gemessen bei einer Scherrate von 10 s⁻¹ bei 25°C, aufweisen
und wobei die vorgemischten Gemische hergestellt werden durch Mischen von (a)
und (b) bei 500-1500 U/min, bis sie bei einer Temperatur von mehr als dem höchsten Schmelzpunkt von irgendeinem vermischten Bestandteil vollständig gemischt sind, und Kühlen auf 20 bis 25°C, um die Vormischung zu bilden.

2. Zusammensetzung nach Anspruch 1, wobei die Vormischungen eine Okklusivität von > 80 aufweisen.

3. Zusammensetzung nach irgendeinem der vorhergehenden Ansprüche, wobei die Vormischung vor dem Kühlen 20-60 Minuten lang bei einer Temperatur von 80°C gemischt wird.

4. Verfahren zum Erhalt von vorgemischten Materialien mit einer Okklusivität von mehr als 80 und einer Viskosität von < 1000 Pa.s bei 10 s⁻¹ bei 25°C, das die Herstellung einer Vormischung, wie durch die Bestandteile (a) und (b) von Anspruch 1 definiert, umfasst.

5. Verfahren nach Anspruch 4, wobei die vorgemischten Materialien hergestellt werden durch Mischen von (a) und (b) bei 500-1500 U/min, bis sie bei einer Temperatur von mehr als dem höchsten Schmelzpunkt von irgendeinem vermischten Bestandteil vollständig vermischt sind, und Kühlen auf 20 bis 25°C, um die Vormischung zu bilden.

6. Verfahren, mit dem Haut oder anderen Substraten eine Befeuchtung und eine Empfindung vermittelt wird, die der (dem) von Petrolat gleichkommt, welches das Auftragen eines speziellen Vorgemischs nach irgendeinem der Ansprüche 1 bis 3 auf das Substrat umfasst.

## Revendications

1. Composition de soin des cheveux ou de lavage pour la personne comprenant une composition de pré-mélange, la composition de pré-mélange comprenant :
(a) 25-95 % en masse de mélange pré-combiné comprenant des liquides hydrocarbonés étant des oligomères et polymères dérivés du pétrole comprenant des groupes monomères alcène ; ou deux ou plusieurs unités alcène ; où lesdits liquides hydrocarbonés présentent un point de fusion ou de transition vitreuse < 30°C et une viscosité de 500 Pa.s ou inférieure à de 20 à 25°C et où les liquides hydrocarbonés sont du polybutène ou du caoutchouc naturel ;
(b) 10 à 75 % en masse de mélange pré-combiné comprenant un matériau structurant choisi dans le groupe constitué de cires ; où ledit matériau structurant présente un point de fusion > 30°C et où la cire est de la cire de Candelilla,
où lesdits mélanges pré-combinés comprennent des matériaux liquides hydrocarbonés fluides (Constituant a) structurés avec des matériaux de b ;
où lesdites pré-combinaisons présentent une viscosité inférieure à 1 000 Pa.s mesurée à une vitesse de cisaillement de 10 s⁻¹ à 25°C
et où lesdits mélanges pré-combinés sont préparés en mélangeant (a) et (b) à 500-1 500 tr/min jusqu'à ce qu'ils soient complètement mélangés à une température supérieure au point de fusion le plus élevé de tout ingrédient étant combiné et en refroidissant à de 20 à 25°C pour former une pré-combinaison.

2. Composition selon la revendication 1, où les pré-combinaisons présentent une occlusivité de > 80.

3. Composition selon l'une quelconque des revendications précédentes, où la pré-combinaison est mélangée à une température de 80°C pendant 20-60 minutes avant refroidissement.

4. Procédé d'obtention de matériaux pré-combinés présentant une occlusivité supérieure à 80 et une viscosité de < 1 000 Pa.s à 10 s⁻¹ à 25°C qui comprend la fabrication d'une pré-combinaison comme définie par des constituants (a) et (b) de la revendication 1.

5. Procédé selon la revendication 4, où lesdits matériaux pré-combinés sont préparés en mélangeant (a) et (b) à 500-1 500 tr/min jusqu'à ce qu'ils soient complètement mélangés à une température supérieure au point de fusion le plus élevé de tout ingrédient étant combiné et en refroidissant à de 20 à 25°C pour former une pré-combinaison.

6. Procédé de fourniture d'une hydratation et d'une sensation semblable au pétrolatum à la peau ou un autre substrat qui comprend l'application à un substrat d'un pré-mélange spécifique selon l'une quelconque des revendications 1 à 3.
